# EUROPEAN PATENT APPLICATION

(11) **EP 1 238 640 A1**
(43) Date of publication of application: **11.09.2002**
(21) Application number: 02290119.3
(22) Date of filing: 17.01.2002
(51) Int. Cl.: A61F 5/01

(54) **Walker brace**

(30) Priority: 09.03.2001 US 274255 P; 06.11.2001 US 985923
(71) Applicant: Darco International, Huntington, WV 25701 (US)
(72) Inventor: Darby, Darrel H., Huntington, WV 25701 (US)
(74) Representative: Busnel, Jean-Benoît

(57) **Abstract**

A novel lower leg, ankle and foot immobilization brace in the form of a short length leg walker. The walker provides controlled, uniform compression of the foot, ankle and leg, while at the same time immobilizing the lower leg and foot. The immobilization walker has a shock absorbing sole, a rigid immobilization plate designed to prevent movement in a patient's ankle and foot, and a flexible boot portion designed to comfortably fit around the patient's foot and lower leg. The immobilization plate and a semi-flexible upper portion effectively immobilize the foot and ankle, while eliminating the need for a rigid boot portion. Two shock absorbing insoles, one of soft density and a one of medium density, allow a portion of the walker's sole to be located within the boot portion.

## Description

### FIELD OF THE INVENTION

The present invention generally relates to braces employed in immobilizing the foot and ankle as a substitute for a rigid cast, and in particular to an improved below the knee walker.

### BACKGROUND OF THE INVENTION

Plaster or synthetic casts have long been recognized as the ideal material for immobilization of the extremities following trauma or surgery. These casts, however, become loose on the leg, ankle, and foot when, after a few days, the edema caused by trauma or surgery subsides. The loosened cast is not only less effective because it no longer provides the necessary compression or immobilization, but is uncomfortable for the wearer.

Furthermore, casts have significant shortcomings for a patient that has an open wound. Casts tend to trap drainage from wounds in the cast padding, creating an ideal breeding ground for bacteria and increasing the chance of a patient's wound getting infected. In addition, casts must be removed in order to treat or cleanse a wound.

A substitute for the cast is the short leg walker immobilization brace. These walkers are generally formed with a rigid rocker sole designed to allow a patient to roll through a gait cycle while immobilizing the ankle, foot and metatarsal-phalangeal joint. Unfortunately, in order to obtain adequate height for rocking, theses walkers have a high profile in many cases. Moreover, the boot portion of these walkers is formed of a rigid material, which can be heavy and uncomfortable.

It is therefore a primary object of the present invention to eliminate the problems commonly associated with casts and walkers and to provide an economical and comfortable device to provide immobilization while allowing the patient to continue ambulation.

### SUMMARY OF THE INVENTION

The present invention is directed to a novel lower leg, ankle and foot immobilization brace in the form of a short length leg walker. The walker provides controlled, uniform compression of the foot, ankle and leg, while at the same time immobilizing the lower leg and foot. The present invention employs a rocker sole to reduce motion, friction, and pressure in the foot and two shock absorbing heat or pressure moldable insoles include a top layer of soft density and a bottom layer of medium density.

The immobilization walker has a shock absorbing sole, a rigid immobilization plate designed to prevent movement in a patient's ankle and foot, and a flexible boot portion designed to comfortably fit around the patient's foot and lower leg. The immobilization plate and a semi-flexible upper portion effectively immobilize the foot and ankle, while eliminating the need for a rigid boot portion. This allows the boot portion of the walker to be constructed of a softer, more flexible material instead of a rigid material, which provides a light weight, comfortable boot.

The boot portion is designed to comfortably fit around the patient's ankle, foot, and lower leg. It has a fixed, vented liner instead of a standard removable liner, and is much softer and more flexible than existing boot portions. The liner replaces the usual bulky, difficult to handle separate liner that is usually hot and uncomfortable This provides a more comfortable fit and allows the walker to be more easily placed on the patient.

Both the boot portion and the immobilization plate have raised vents with air channels that trap air when a patient's leg moves forward forcing air into the vents. These vents also give the patient better ventilation by providing a means for body heat to escape.

In existing walkers, sometimes the buckle snaps open when excess pressure is applied. The buckles on this new walker lock to prevent the buckle from snapping open. Furthermore, in existing braces, sometimes the excess strap protrudes out. This walker has a strap guide on the opposite side of the walker, which causes the excess strap to curve around the boot portion.

### BRIEF DESCRIPTION OF THE DRAWINGS

The advantages, nature and various additional features of the invention will appear more fully upon consideration of the illustrative embodiment of the invention which is schematically set forth in the drawings, in which:
Figure 1 is a lateral view of the immobilization walker (adjustable tension buckle with lock side);
Figure 2 is a lateral view that shows the side opposite to Figure 1 (strap fastener insert side);
Figure 3 is a front view of the immobilization walker, showing closure straps with strap ratchet insert and the adjustable tension buckle with lock and tongue;
Figure 4 is a posterior or back view with leg portion of the immobilization walker ;
Figure 5 is a partial cross sectional view of the immobilization walker showing the vented liner;
Figure 6A is a lateral view of the rigid foot-ankle immobilization plate;
Figure 6B is a top view of the immobilization plate;
Figure 6C is a back view of the immobilization plate;
Figure 7A is a cross sectional view of Figure 1 taken along line 7A-7A;
Figure 7B is a cross sectional view of Figure 1 taken along line 7B-7B;
Figure 7C is a cross sectional view of Figure 1 taken along line 7C-7C;
Figure 8A is a top view of the adjustable tension closure buckle with lock in which the buckle is locked into place;
Figure 8B is a side view of the adjustable tension closure buckle with lock in which the buckle is locked into place;
Figure 8C is a side view of the adjustable tension closure buckle with lock in which the buckle is open;
Figure 8D is a bottom view of the adjustable tension closure buckle with lock in which the buckle is locked into place;
Figure 9A is a top view of the strap with ratchet-type buckle; and
Figure 9B is a side view of the strap with ratchet-type buckle.

### DETAILED DESCRIPTION OF THE INVENTION

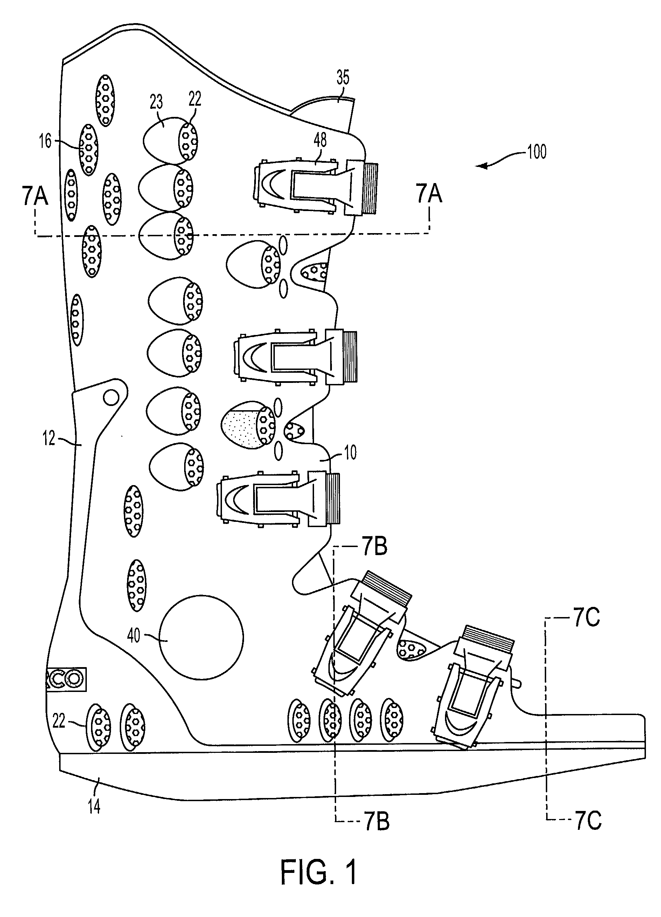

The improved short leg walker brace 100 provides immobilization and uniform, adjustable compression, which prevents torsion of a patient's leg, ankle and foot as well as dorsi-flexion and plantar flexion at the ankle joint. Other advantages of the present invention will become apparent from the description of the preferred embodiments and from the accompanying drawings. Referring to Figure 1, the invention is a lower leg, ankle and foot, immobilization walker 100 with an boot portion 10, a rigid immobilization plate 12, and an outer sole 14. The pictured embodiment is a high top walker 100, but a low top model may be used if appropriate for the patient's injury.

The boot portion 10 is formed of a semi-flexible material. In this embodiment, the boot portion is made of an injection molded material such as polyurethane, but it can be made of any semi-flexible material such as leather. The boot portion 10 surrounds and supports an internal, breathable foam liner 16. The walker 100 provides controlled, uniform compression of a patient's foot, ankle and leg covered by the brace, while at the same time immobilizing these parts of the body.

The boot portion 10 has a number of ventilation openings 22, some of which are raised openings with air channels 23 that extend out from the sides. The raised channels 23 catch air as the leg moves forward forcing the air through the channel and vented liner 16 to cool the foot and leg and allow body heat to escape.

The boot portion 10 also has convex areas 40 on both sides corresponding to the location of the medial and lateral ankle malleoli. These areas 40 permit a better fitting walker 100 and reduce the possibility of pressure on these ankle prominences.

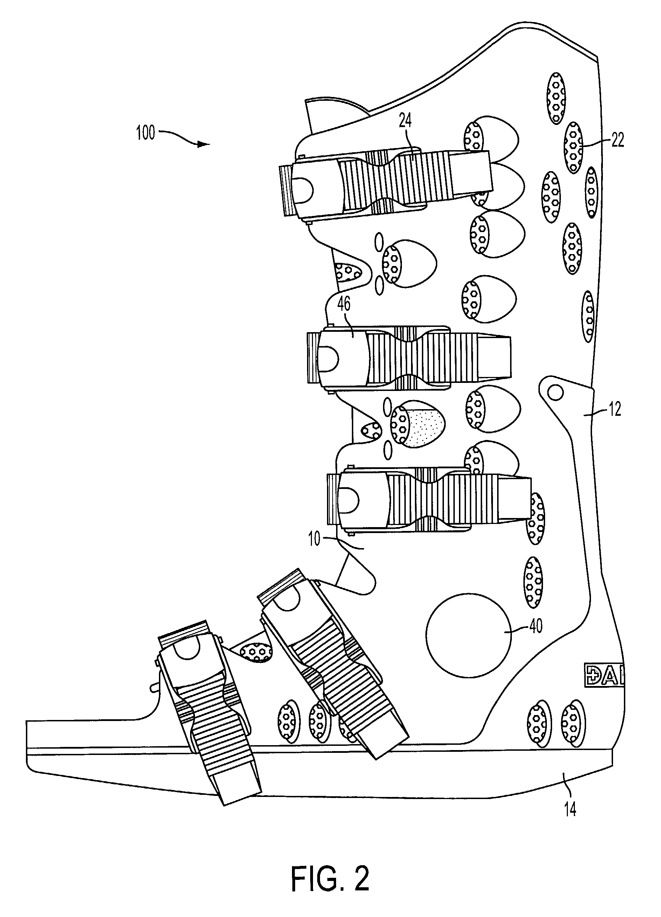

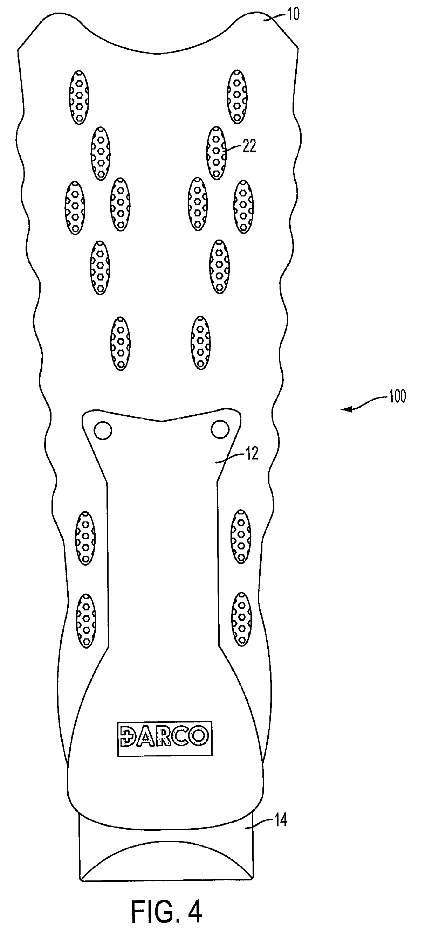

Figures 2-4 are additional views of the walker 100.

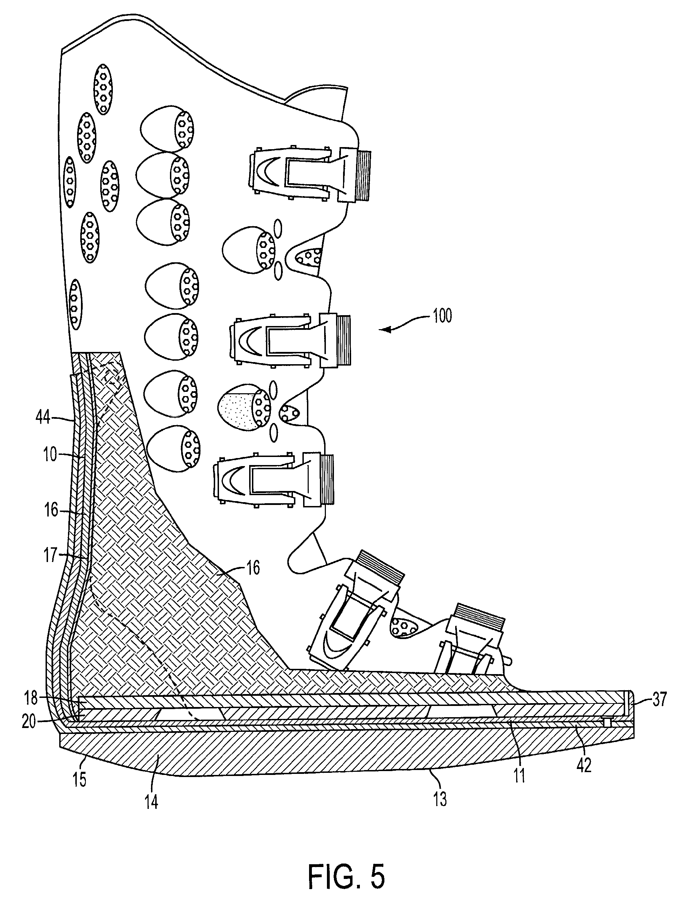

Turning to Figure 5, the already introduced liner 16 is made of breathable, open pore foam of soft ethylene vinyl acetate ("EVA") or other suitable material. The liner 16 is covered with a loose weave absorbable fabric 17. The liner 16 reduces friction and pressure and generally provides a comfortable fit to the lower leg and foot. The liner 16 may be glued directly to the boot 10 to forms a principal element of the improved immobilization walker. This eliminates the necessity of a separate liner with hook and loop closures which becomes difficult for patients to get on and off. The foam liner 16 is breathable, which provides ventilation to a patient's and reduces the overall weight of the short length leg walker 100.

The rigid right-angled foot-ankle stabilization plate 12 is attached and formed outside the boot portion 10. The plate 12 has a bottom portion 42 that covers a foot section 11 of the boot portion 10 and a back portion 44 that preferably extends about half way up the back of the walker. The foot-ankle stabilization plate 12 is preferably attached to the boot 10 at a pre-molded area by glue or rivets.

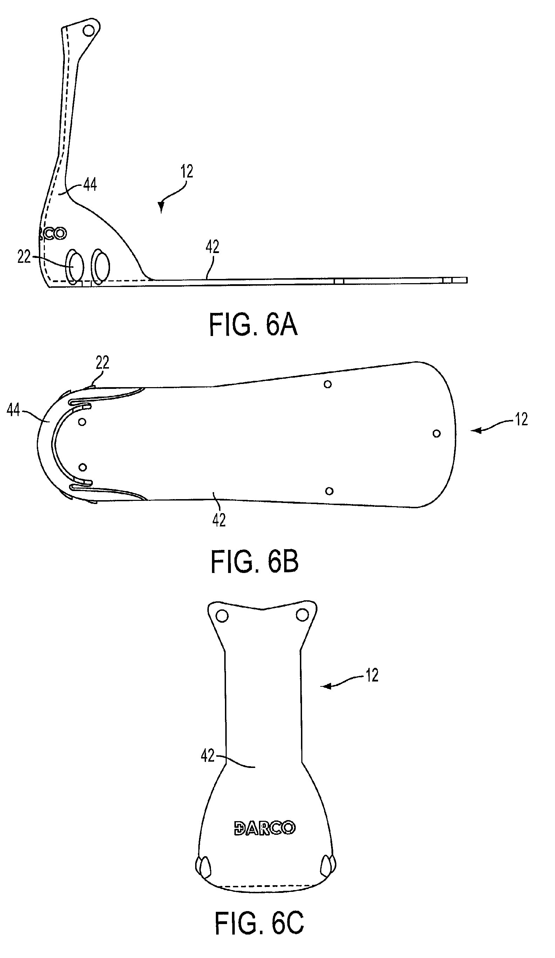

The immobilization plate is shown in detail in Figures 6A, 6B, and 6C. Note that the back portion 44 of the plate 12, like the boot portion 10, is provided with ventilation ports 22 to ventilate the leg, ankle and foot of the user by both allowing cool air to enter the boot and warm air to escape.

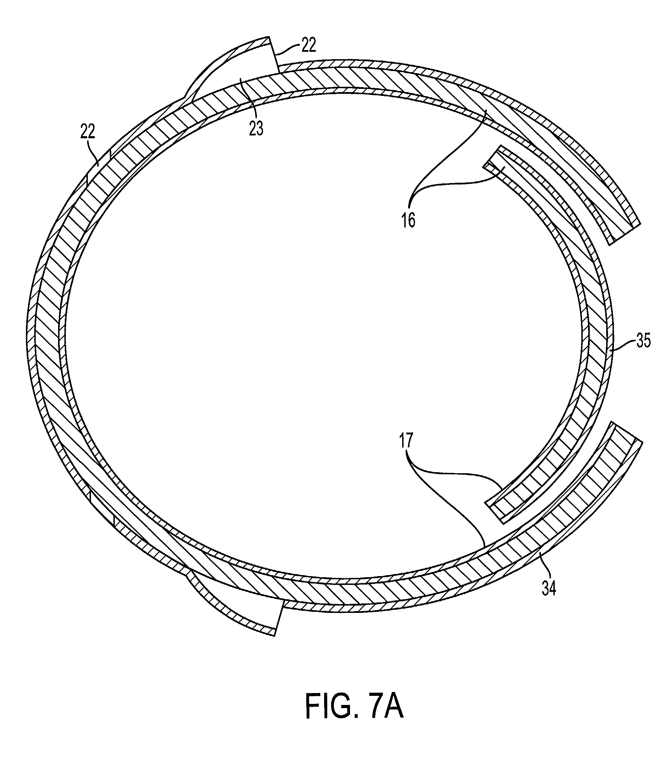

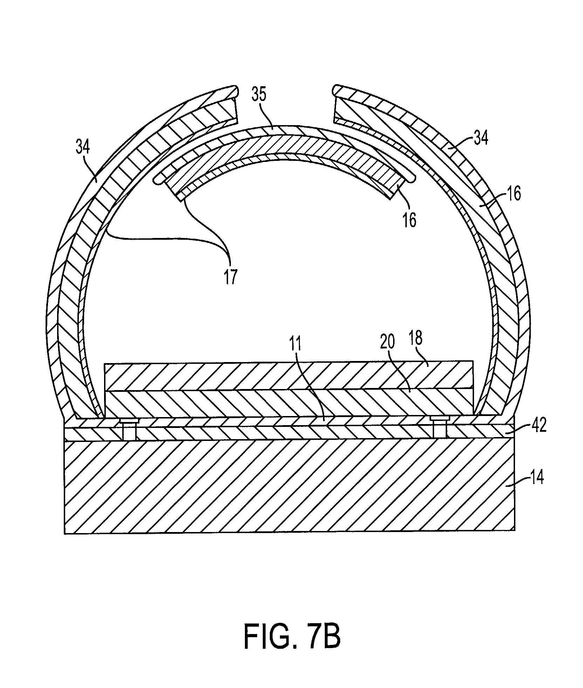

Turning to Figures 7A and 7B, the boot portion 10 that surrounds the patient's foot has generally U-shaped section 34. An open front between the sides of the U-shaped section 34 extends over the complete length of the immobilizing walker 100 and allows a patient to place her heel and foot into the brace. The boot 10 also has a tongue portion 35 that is constructed of the same material as the remaining boot 10. The tongue 35 has vents 22 and a liner 16.

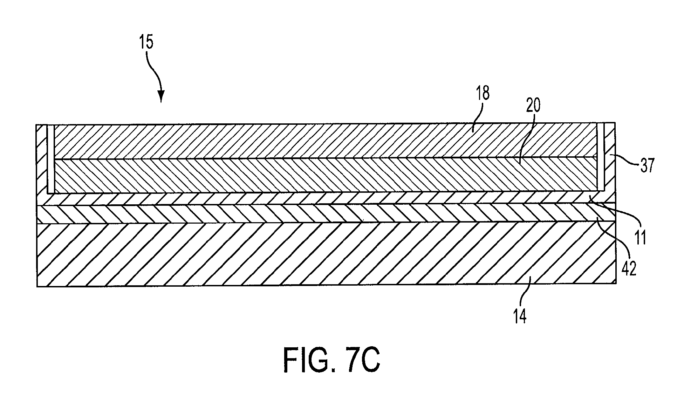

As shown in Figures 5 and 7C, an extension 37 of the boot portion 10 at the location of the patient's toe and the lower part of the boot portion 10 located around the rest of the patient's foot form a circumferential counter that surrounds the location where the patient's foot is located. The extension allows the patient's toes to be exposed. Note that this extension 37 surrounds the front of the toes, as shown in Figure 5, and the sides of the toes as shown in Figure 7C. The circumferential counter, the extension 37 and the lower part of the boot portion 10, defines a vertical rim around a patient's foot that forms a pocket that holds removable insoles 18, 20, which are discussed below.

Referring to Figure 7C, the walker 100 is provided with a walker sole 15 that is a combination of various parts including a shock absorbing outer sole 14, the bottom portion 42 of the immobilization plate 12. the foot portion 11 of the boot portion 10, and the two insoles 18, 20. The outer sole 14, which has a significant thickness, is bonded to the top of the bottom portion 42 of the ankle immobilization plate 12. The bottom portion of the plate 42 is bonded to the foot portion 11 of the boot portion 10. Two insoles 18, 20, which have different densities, rest on top of the foot portion 11 to complete the thickness of the walking sole 15.

Referring back to Figure 5, the insoles 18, 20 allow a portion of the thickness of the walker sole 15 to be inside the walker 100, thus providing a lower profile to the walker sole. This provides a highly stable, weight bearing surface. In addition, the walker sole 15 is of reduced bulk compared to existing walker soles which have significantly greater thickness. The thickness of the walking sole 15 is approximately the same thickness as a conventional shoe or boot sole.

The bottom surface 13 of the rigid outer sole 14 has a curved upward heel portion, a central portion that it relatively flat to provide stability, and a toe portion that curves upwardly again at the apex of the ball area terminating at the end of the toe portion of the shoe. Over its length, the upper surface of the outer sole 14 is preferably contoured to provide a better fit for the user's foot with good stability and comfort.

The foot portion 11 of boot 10 is contoured much like conventional footwear with a rounded heel counter and a contoured recessed foot bed that holds the pressure moldable shock absorbing, foam insoles 18, 20. This provides a better fit with greater stability and comfort to the patient. The outer sole 12 and the foot portion 11 both narrow from heel to toe in front of the ankle, widen in the vicinity of the metatarsal-phalangeal joint, and further narrow slightly at the toe of the immobilization brace, as would a standard shoe.

The shock absorbing foam insoles 18, 20 function to pad the plantar surface of the user's foot and to provide comfort and protection to the same. The insoles may be formed of EVA foam or other suitable material. Two removable insoles 18, 20 are used. The upper insole 18 is made of a soft density EVA, and the lower insole 20 of a medium density EVA. The insoles 18, 20 are pressure moldable and conform to the plantar aspect of the foot providing total contact with the sole of the foot and more comfort to the patient. These multi-density insoles 18, 20 allow the walker 100 to be used as a total contact cast in the treatment of diabetic ulcers, pressure points, and other lesions of the foot. The lower medium density insole 20 may be altered by cutting out an area under a lesion or pressure point to off load or re-distribute body weight away from the lesions or specific areas of pressure on the plantar surface of the foot.

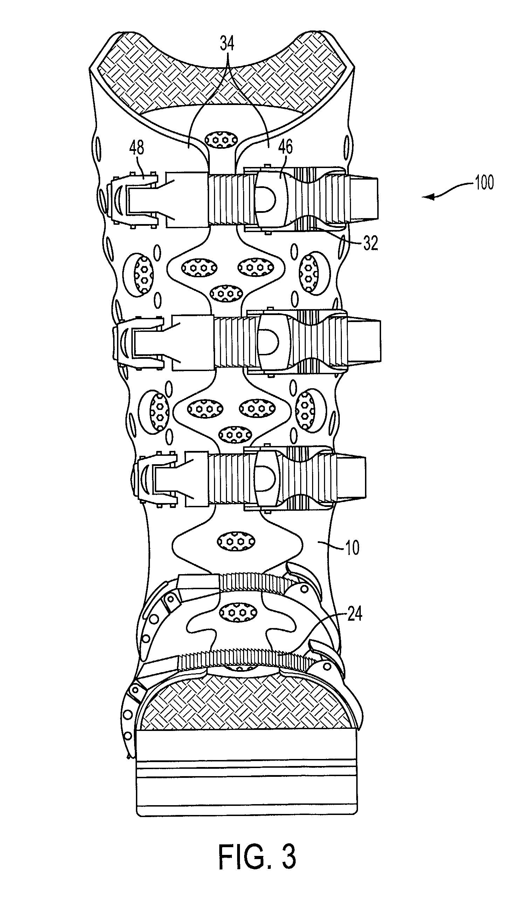

Turning back to Figure 3, the boot portion 10 has several adjustable straps 24 that allow the walker 100 to provide compression to a patient's foot, ankle, and lower leg. A number of adjustable self-locking tension closure buckles 48 are attached to notches at several locations along one side of the U-shaped section 34 of boot portion 10. An equal number of ratchet type buckles are attached to the other side of the U-shaped section 34.

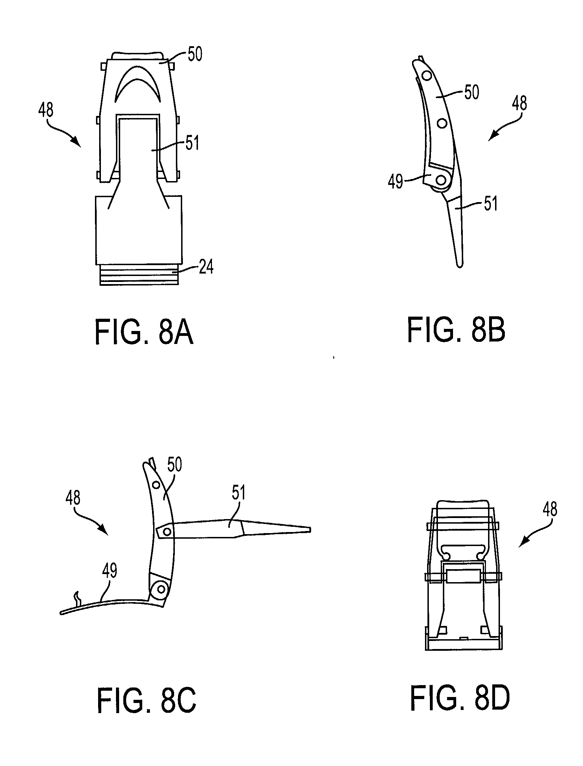

As shown in Figures 8A-8D, each tension closure buckle 48 has a base portion 49 that is attached to the boot portion 10, a locking portion 50 that is attached to the base portion, and a strap portion 51 that is attached to the locking portion. Serrated straps 24 are attached to the strap portions 51.

To tighten the straps 24, the straps 24 are taken across the opening between the two sides of the U-shaped portion 34 and inserted into the ratchet type buckles 46. The locking portion 50 of the tension closure buckle 48 is in an open position, as shown in Figure 8C, at this time.

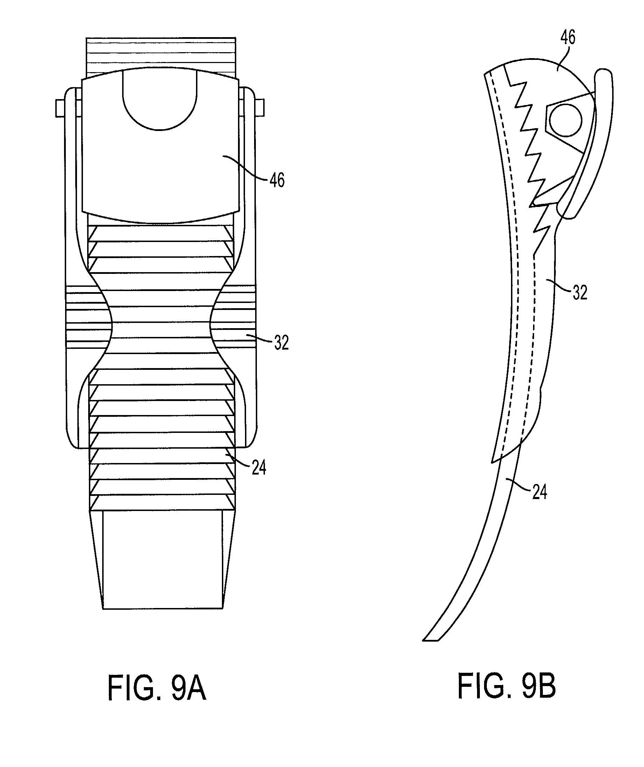

As shown in Figures 9A and 9B, the ratchet type buckles 46 grip the serrations of the straps 24 to allow the straps 24 to be connected to both sides of the U-shaped portion 34 and to lock the strap at a desired length. A strap guide 32 on the ratchet type buckle allows the excess strap 24 to curve around the boot portion 10 and prevents the excess strap 24 from protruding out.

As shown in Figure 8B, the locking portion 50 of tension closure buckle 48 is then closed and locked to the base portion 51. The tension buckle 48, as it is being closed, draws the opposite sides of the U-shaped portion 34 of the boot portion 10 toward each other to compress the patient's encased limb at the desired and comfortable compression level. The amount of compression is adjusted by changing the location where the strap 24 is gripped by the ratchet-type buckle 46.

The straps 24 can be tightened or loosened as dictated by the patient's condition insuring a perfect fit throughout the healing process. In addition, these straps allow the walker 100 to be easily removed for bathing, cleansing and dressing the wound if there is such a need due to surgery or a break in the skin due to trauma. The straps 24 allow the walker 100 to provides uniform compression over the leg, foot, and ankle areas. The large number of straps 24 allow the walker to provide compression that is focal in nature by adjusting the compression of straps 24 over the totality of the walker 100.

The adjustable straps 24 can also be tightened or loosened by the patient to accommodate an increase or decrease in edema on a localized basis. As a result, the improved short length leg walker of the invention is readily adjustable in terms of applied compression uniformly over the foot to compensate for either increased or decreased edema and assuring a good fit of the walker throughout the healing process

It is of course understood that departures can be made from the preferred embodiment of the invention by those of ordinary skill in the art without departing from the spirit and scope of the invention that is limited only by the following claims. For example, the invention is not limited to the described materials, but different materials can be used for the various parts if they are able to function in much the same way as the described materials; and alternative strap arrangements can be used, such as a conventional belt-type buckle in combination with as strap with holes, as long as the alternative strap arrangement allows the strap to be locked into place.

## Claims

1. An immobilization walker comprising:
an outer sole (14) with a back end;
a rigid immobilization plate (12) including:
a back portion (44) that extends up from the back end of the outer sole (14), and
a bottom portion (42) attached to the top of the outer sole: and
a boot portion (10) attached to the rigid immobilization plate (12);
wherein the rigid immobilization plate (12) is designed to prevent movement in a patient's ankle and the boot portion (10) is designed to fit around the patient's foot and lower leg.

2. The immobilization walker of claim 1, wherein the boot portion (10) has at least one ventilation port (22).

3. The immobilization walker of claim 2, wherein the ventilation port (22) is raised.

4. The immobilization walker of claim 1, wherein the rigid immobilization plate (12) has at least one ventilation port (22).

5. The immobilization walker of claim 4, wherein the ventilation port (22) is raised.

6. The immobilization walker of claim 1, wherein a liner (16) is fixed inside the boot portion (10).

7. The immobilization walker of claim 6, wherein the liner (16) is made of a soft, breathable material.

8. The immobilization walker of claim 1, wherein the boot portion (10) is made of a semi-flexible material.

9. The immobilization walker of claim 8, wherein the semi-flexible material is polyurethane.

10. The immobilization walker of claim 1, wherein the boot portion (10) is located inside the immobilization plate (12).

11. An immobilization walker comprising:
an outer sole (14) with a back end;
a rigid immobilization plate (12) including:
a back portion (44) that extends up from the back end of the outer sole (14), and
a bottom portion (42) attached to the top of the outer sole (14), wherein the bottom portion has a top;
a boot portion (10) attached to the top of the bottom portion (42) of the rigid immobilization plate (12);
a lower removable insole (20) made of a material with a density; and
an upper removable insole (18) made of a material with a softer density than the lower insole (20);
wherein the rigid immobilization plate (12) is designed to prevent movement in a patient's ankle and the boot portion (10) is designed to fit around the patient's foot and lower leg, and
the lower removable insole (20) is located on top of the boot portion (10), and the upper removable insole (18) is located on top of the lower removable insole (20).

12. The immobilization walker of claim 11, wherein the outer sole (14) comprises a curved heel portion, a flat central bottom surface portion, and a curved toe portion.

13. The immobilization walker of claim 11, wherein the boot portion further comprises a circumferential counter for providing a pocket for the removable insoles (18, 20).

14. The immobilization walker of claim 11, wherein the boot portion (10) includes a foot portion (11) that is attached to the top of the bottom portion (42) of the rigid immobilization plate (12); and
wherein a combined thickness of the removable insoles (18, 20), the foot portion (11), the bottom portion (42) of the rigid immobilization plate (12), and the outer sole (14) is approximately equal to a thickness of a conventional boot.

15. The immobilization walker of claim 11, wherein at least one of the boot portion (10) and the rigid immobilization plate (12) has at least one raised ventilation port (22).

16. The immobilization walker of claim 11, wherein a liner (16) is fixed inside the boot portion (10).

17. The immobilization walker of claim 16, wherein the liner (16) is made of a soft, breathable material.

18. The immobilization walker of claim 11, wherein the boot portion is made of a semi-flexible material.

19. An immobilization walker comprising:
an outer sole (14) with a back end;
a rigid immobilization plate (12) including:
a back portion (44) that extends up from the back end of the outer sole (14), and
a bottom portion (42) attached to the top of the outer sole (14),
a boot portion (10) attached to the rigid immobilization plate (12), wherein the boot portion has a first side separated by an open front from a second side;
a strap (24) attached to boot portion (10) on the first side of the boot portion,
a means (48) for attaching the strap (24) attached to the second side of the boot portion; wherein the rigid immobilization plate (12) is designed to prevent movement in a patient's ankle,
the boot portion (10) is designed to fit around the patient's foot and lower leg,
the means (48) for attaching the strap has an attaching lock portion (50),
the strap spans the open front, and
the strap (24) is attached to the means (48) for attaching the strap and is tightened to provide compression to the patient's at least one of ankle, foot, and lower leg.

20. The immobilization walker of claim 19, further comprising a means (46) for tightening the strap (24), wherein the means for tightening the strap provides the tightening of the strap.

21. The immobilization walker of claim 20, wherein the means (46) for tightening the strap has a lock portion.

22. The immobilization walker of claim 21, wherein the means (46) for tightening the strap is attached to the second side of the boot portion (10).

23. The immobilization walker of claim 22, wherein the means for tightening the strap is a first buckle (46).

24. The immobilization walker of claim 23, wherein the means for attaching the strap is a second buckle (48).

25. The immobilization walker of claim 24, further comprising a strap guide (32) attached to the second side of the boot portion, wherein the strap guide causes a part of the strap to curve around the boot portion.

26. The immobilization walker of claim 19, wherein the means for attaching the strap provides the tightening of the strap.

27. The immobilization walker of claim 25, wherein the means for attaching the strap is a buckle.

28. The immobilization walker of claim 26, further comprising a strap guide (32) attached to the second side of the boot portion, wherein the strap guide causes a part of the strap that is not tightened to curve around the boot portion.
